(19) Europäisches Patentamt / European Patent Office / Office européen des brevets

(11) **EP 4 674 442 A1**

(12) **EUROPEAN PATENT APPLICATION**
published in accordance with Art. 153(4) EPC

(43) Date of publication:
07.01.2026 Bulletin 2026/02

(21) Application number: 24825897.2

(22) Date of filing: 18.06.2024

(51) International Patent Classification (IPC):
A61L 15/24 (2006.01)    A61F 13/02 (2024.01)
A61K 9/70 (2006.01)     A61K 47/32 (2006.01)
A61K 47/34 (2017.01)    A61P 17/02 (2006.01)

(52) Cooperative Patent Classification (CPC):
A61F 13/02; A61K 9/70; A61K 47/32; A61K 47/34;
A61L 15/24; A61P 17/02

(86) International application number:
PCT/JP2024/022052

(87) International publication number:
WO 2024/262496 (26.12.2024 Gazette 2024/52)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 19.06.2023 JP 2023100208

(71) Applicant: Japan Exlan Company Limited
Okayama-shi, Okayama 704-8510 (JP)

(72) Inventors:
• KOMIYAMA Takuzo
  Okayama-shi Okayama 704-8510 (JP)
• SHIMIZU Haruki
  Okayama-shi Okayama 704-8510 (JP)

(74) Representative: Patentanwälte
Ruff, Wilhelm, Beier, Dauster & Partner mbB
Kronenstraße 30
70174 Stuttgart (DE)

(54) **MATERIAL FOR PROMOTING RELEASE OF OXYGEN FROM RED BLOOD CELLS, AND WOUND COVERING MATERIAL CONTAINING SAID MATERIAL**

(57) It is known that during the wound healing process, when pH in blood decreases, oxygen is released from hemoglobin, blood oxygen concentration rises, division of regenerated epithelial cells, and healing is promoted . Wound healing agents such as creams and ointments are generally weakly acidic, and therefore can be expected to lower pH in blood and to promote wound healing. However, the wound healing agents such as creams and ointments are inferior in terms of workability, and there is a risk of presenting further damage to the wound when applied. An object of the present invention is to provide a material for promoting oxygen release from red blood cells, which has excellent workability and can promote division of regenerated epithelial cells in order to facilitate the wound healing. The material for promoting oxygen release from red blood cells according to the present invention is characterized in that the material for promoting oxygen release from red blood cells is a sheet-like material containing an acid-type carboxyl group-containing material, wherein the amount of acid-type carboxyl groups contained in the sheet-like material is 0.05 mmol/g or more and 10.0 mmol/g or less.

## Description

Technical Field

The present invention relates to a material for promoting oxygen release from red blood cells and a wound dressing containing the same.

Background Art

Physical damage to body surface tissues caused by external or internal factors is called a wound. A hygienic material placed to cover the surface of such a wound (wound surface) is called a wound dressing, and it plays roles of protecting the wound surface, preventing drying (moisturizing), absorbing exudates (liquid absorption), and inhibiting bacterial infection. Compared with the conventional mainstream method of dressing the wound surface with gauze and disinfecting it with an antiseptic solution, wound dressings maintain a moist state on the wound surface, so that cell activation is not inhibited. Therefore, wood dressings enable effective wound healing. Various wound dressings have been reported (e.g., Patent Documents 1 and 2).

On the other hand, it is known that during the wound healing process, an increase in oxygen concentration in blood promotes division of regenerated epithelial cells, thereby promoting wound healing (Non-Patent Document 1). The binding strength between hemoglobin and oxygen in blood decreases as pH in blood decreases, so this phenomenon can be utilized to increase oxygen concentration in blood. From this perspective, wound healing agents such as creams and ointments, which are generally weakly acidic, can be expected to lower pH in blood and to promote wound healing.

Prior Art Documents

Patent Documents

    Patent Document 1: Japanese Patent Application Laid-Open (JP-A) No. 201838/98
    Patent Document 2: Japanese Patent Application Laid-Open (JP-A) No. 2000-210375

Non-patent Documents

Non-Patent Document 1: Aichi Prefectural journal of hospital pharmacy, 2005, Vol. 33, No. 3, p. 6-16

However, the wound healing agents such as creams and ointments is not necessarily the best choice. For example, the wound healing agents such as creams and ointments need to be applied by fingers, which brings about a need to wipe off the fingers after the application. Therefore, the wound healing agents such as creams and ointments are inferior in terms of workability. Also, there is a risk of presenting further damage to the wound when applied.

Disclosure of the Invention

Problem that the Invention is to solve

The present invention has been made in consideration of the above circumstances. An object of the present invention is to provide a material for promoting oxygen release from red blood cells, which has excellent workability and can promote division of regenerated epithelial cells in order to facilitate the wound healing.

Means for solving the problem

The inventors of the present invention have conducted an extensive research for achieving the above-mentioned object. As a result, the inventors of the present invention have found that an acid-type carboxyl group-containing material can be used as a material for promoting oxygen release from red blood cells, which have excellent workability in wound healing. The inventors of the present invention have thus completed the present invention.

That is, the present invention is achieved by the following means.

    (1) A material for promoting oxygen release from red blood cells, characterized in that the material for promoting oxygen release from red blood cells is a sheet-like material containing an acid-type carboxyl group-containing material, wherein the amount of acid-type carboxyl groups contained in the sheet-like material is 0.05 mmol/g or more and 10.0 mmol/g or less.

(2) The material for promoting oxygen release from red blood cells according to (1), wherein the amount of acid-type carboxyl groups in the acid-type carboxyl group-containing material is more than 1.5 mmol/g and not more than 10.0 mmol/g.

(3) The material for promoting oxygen release from red blood cells according to (1), wherein the content of the acid-type carboxyl group-containing material is 1 to 100% by mass.

(4) The material for promoting oxygen release from red blood cells according to (1), wherein the acid type carboxyl group-containing material has a fibrous, particulate or film form.

(5) The material for promoting oxygen release from red blood cells according to (1), wherein the acid-type carboxyl group-containing material is a sheath-core composite fiber consisting of a sheath portion and a core portion, wherein the sheath portion contains an acid-type carboxyl group-containing polymer, and wherein the core portion contains an acrylonitrile-based polymer.

(6) The material for promoting oxygen release from red blood cells according to any one of (1) to (5), wherein the material for promoting oxygen release from red blood cells is in the form of nonwoven fabric, woven fabric, knitted fabric, paper or film.

(7) A wound dressing containing the material for promoting oxygen release from red blood cells according to (6).

(8) The wound dressing according to (7), wherein a water vapor permeable material is laminated to the material for promoting oxygen release from red blood cells.

(9) The wound dressing according to (8), wherein the water vapor permeable material is in the form of silicone or polyurethane film.

Effect of the Invention

The material for promoting oxygen release according to the present invention can efficiently release oxygen from red blood cells. Furthermore, the acid-type carboxyl group-containing material of the present invention has been widely used in clothing and other materials, and various processing methods have been established, so it can be widely used as a wound dressing.

Mode for carrying out the Invention

The material for promoting oxygen release of the present invention is a sheet-like material containing an acid-type carboxyl group-containing material. The amount of acid-type carboxyl groups contained in the material for promoting oxygen release of the present invention is 0.05 mmol/g to 10.0 mmol/g, preferably 0.2 mmol/g to 8.0 mmol/g, relative to the mass of the material for promoting oxygen release. If the amount of acid-type carboxyl groups is less than 0.05 mmol/g, oxygen release from red blood cells may not be sufficiently promoted. On the other hand, if the amount of acid-type carboxyl groups is more than 10.0 mmol/g, production at a practical level may be difficult, and even if production is possible, it may be difficult to achieve satisfactory physical properties. In the present invention, an acid-type carboxyl group refers to "-COOH," and a salt-type carboxyl group refers to one in which a hydrogen ion of an acid-type carboxyl group is replaced with other ion.

In the acid-type carboxyl group-containing material used in the material for promoting oxygen release of the present invention, the amount of acid type carboxyl groups is preferably more than 1.5 mmol/g and not more than 10.0 mmol/g, more preferably more than 2.0 mmol/g and not more than 10.0 mmol/g, even more preferably more than 5.0 mmol/g and not more than 10.0 mmol/g, and most preferably more than 5.0 mmol/g and not more than 8.0 mmol/g. If the amount of acid-type carboxyl groups is 1.5 mmol/g or less, oxygen release from red blood cells may not be sufficiently promoted. If it is more than 10.0 mmol/g, it may be difficult to produce at a practical level, and even if it can be produced, it may not be possible to process it due to unsatisfactory physical properties.

The material for promoting oxygen release of the present invention preferably contains the acid type carboxyl group-containing material in an amount of 1 to 100% by mass, more preferably 3 to 50% by mass, and even more preferably 5 to 30% by mass. If the amount is less than 1% by mass, oxygen release from red blood cells may not be sufficiently promoted.

The acid type carboxyl group-containing material may be in the form of fibers, particles, films, or the like. In the case of fibers, there are a wide variety of processing methods available for imparting the desired form, making it easy to achieve to various forms. Furthermore, during such processing, there is no need to use a surface-coating agent such as a binder so that the deterioration of the oxygen release-promoting function due to coating can be avoided.

The acid-type carboxyl group-containing material may be constituted at least partially from an acid-type carboxyl group-containing polymer. Specific examples of such an acid-type carboxyl group-containing polymer include polyacrylic acid, polymethacrylic acid, polymaleic acid, and copolymers of acrylic acid, methacrylic acid, and maleic acid. Also, usable are products obtained by crosslinking water-soluble vinyl polymers having acid-type carboxyl groups with a crosslinking compound, as disclosed in JP-A-2009-173690.

Examples of the fibrous acid-type carboxyl group-containing material include fibers obtained by an acid-treatment of crosslinked acrylate fibers obtained by subjecting acrylate fibers to a crosslinking introduction treatment using a crosslinking agent such as hydrazine and to a hydrolysis treatment using an alkaline compound; and fibers obtained by the method described in JP-A-2013-147774.

Examples of the crosslinked acrylate fibers include crosslinked acrylate fibers obtained by crosslinking acrylic fibers having an acrylonitrile content of 85 to 95% by weight with a hydrazine compound, in which an increase in a content of nitrogen induced by the crosslinking treatment is 1.0 to 5.0% by weight, and in which a portion of the remaining nitrile groups are converted to alkali metal salt-type carboxyl groups of 3.0 to 6.0 meq/g by hydrolysis treatment, as described in JP-A-2000-314082. Such crosslinked acrylate fibers can be used after converting the salt-type carboxyl groups to acid-type carboxyl groups by, for example, immersing the crosslinked acrylate fibers in a nitric acid aqueous solution. The amount of acid-type carboxyl groups can be adjusted by changing the agent concentration, reaction time, reaction temperature, etc. in hydrolysis treatment.

Furthermore, when a sheath-core composite fiber consisting of a sheath portion and a core portion, wherein the sheath portion contains an acid-type carboxyl group-containing polymer, and wherein the core portion contains an acrylonitrile-based polymer is used as the acid-type carboxyl group-containing material having a fibrous form, the degradation of physical properties of fibers caused by increasing the amount of acid-type carboxyl groups can be suppressed, thereby achieving both the function of promoting oxygen release from red blood cells and physical properties of fiber. Examples of such a sheath-core composite fiber include one obtained by subjecting the fiber surface of an acrylonitrile-based fiber to the aforementioned crosslinking and hydrolysis treatments so as to produce a metal salt-type acrylate-based fiber, followed by immersion in a nitric acid aqueous solution so as to convert the salt-type carboxyl groups to acid-type carboxyl groups, as described in JP-A-2023-67820, and commercially available products in which the salt-type carboxyl groups have been converted to acid-type carboxyl groups. Examples of such a commercially available product include the acrylonitrile-based absorbent sheath-core fiber "LANSEAL" manufactured by Toyobo Co., Ltd., which has a sheath portion containing a salt-type carboxyl group-containing polymer and a core portion containing an acrylonitrile-based polymer. Furthermore, "LANSEAL" has excellent water absorbability, and when used as a wound dressing, it can absorb sweat, exudates, etc., so that it can maintain moisturized state of the wound and heal the wound effectively.

Examples of particulate acid-type carboxyl group-containing materials include cation exchange resins and crosslinked particles containing acid-type carboxyl groups that can be produced by the method described in JP-A-2013-147473.

The form of the material for promoting oxygen release of the present invention is not particularly limited, and is preferably in the form of a sheet such as nonwoven fabric, woven fabric, knitted fabric, paper, or film. The material for promoting oxygen release in the sheet form is easily used as a wound dressing as described below.

In the method for producing the material for promoting oxygen release of the present invention, when the acid-type carboxyl group-containing material is in the form of fiber, it is preferable to use the material alone or together with other fiber materials so as to form nonwoven fabric, woven fabric, knitted fabric, paper, or the like according to a conventionally known method. The other fiber materials are not particularly limited, and natural fibers, organic fibers, semi-synthetic fibers, and synthetic fibers can be used, and inorganic fibers, glass fibers, or the like can also be used depending on the application. Specific examples thereof include cotton, linen, silk, wool, nylon, rayon, polyester, and acrylic fibers.

When the acid type carboxyl group-containing material is in a particulate form, it can be supported on the surface of nonwoven fabric, woven fabric, knitted fabric, paper, film, or the like made of other materials using a binder or the like.

The material for promoting oxygen release of the present invention described above is suitable as a material for constituting a wound dressing. That is, the material for promoting oxygen release of the present invention can efficiently release oxygen from red blood cells, and when used as a wound dressing, the released oxygen promotes division of regenerated epithelial cells and facilitates the wound healing. Furthermore, it can be in the form of nonwoven fabric, woven fabric, knitted fabric, paper, or film, so that, unlike ointments, it does not require wiping off and has excellent workability.

Although the material for promoting oxygen release of the present invention can be used alone as a wound dressing, it becomes even more suitable as a wound dressing when a water vapor permeable material is laminated to the material for promoting oxygen release. That is, by laminating a water vapor permeable material on the side opposite the wound surface, it is possible to protect the wound surface from external contamination such as bacteria and external moisture while allowing moisture derived from exudates from the wound surface and insensible perspiration to dissipate appropriately.

The water vapor permeable material is not limited, and examples thereof include silicone film and polyurethane film. They are flexible and thus can easily conform to the shape of the wound surface.

As to a method for laminating and integrating a water vapor permeable material onto the material for promoting oxygen release of the present invention, it can be mentioned to laminate and integrate the water vapor permeable material onto the material for promoting oxygen release via a conventionally known adhesives or glues that have water vapor permeability.

Examples

Examples are described below in order to facilitate understanding of the present invention, but these are merely illustrative and the present invention is not limited thereto. In Examples, parts and percentages are expressed by mass unless otherwise specified.

<Measurement of the amount of acid-type carboxyl groups>

Approximately 1 g of a thoroughly dried sample was weighed out (W1 [g]), 200 ml of water was added to this, and a titration curve was obtained using 0.1 mol/l aqueous sodium hydroxide solution according to a standard method. From the titration curve, the amount of aqueous sodium hydroxide consumed by the acid-type carboxyl groups (V1 [ml]) was determined, and the amount of acid-type carboxyl groups was calculated using the following formula:

$$\text{Amount of acid-type carboxyl groups [mmol/g]} = 0.1 \times V1/W1$$

<Measurement of pH of horse blood after treatment>

An evaluation sample (0.3 g) was added to horse blood (sold by Japan Bioserum Co., Ltd.) and allowed to stand at room temperature for 5 minutes. The evaluation sample was then removed and pH in the horse blood was measured.

<Oxygen Release Rate>

Hemoglobin bound to oxygen (oxyhemoglobin) and hemoglobin not bound to oxygen (deoxyhemoglobin) have different three-dimensional structures from each other, and therefore also have different infrared absorption curves from each other. Specifically, the relationship between the absorbance at a wavelength of 665 nm (A665) and the absorbance at a wavelength of 880 nm (A880) is such that A665<A880 for oxyhemoglobin, whereas A665>A880 for deoxyhemoglobin. Utilizing this relationship, the oxygen release rate was evaluated by the following method.
An evaluation sample (0.3 g) was added to horse blood (sold by Japan Bioserum Co., Ltd.) and allowed to stand at room temperature for 5 minutes. The evaluation sample was then removed and the horse blood was collected and diluted 100 times with physiological saline solution. Then, A665 and A880 were measured and the oxygen release rate was calculated using the following formula. If the oxygen release rate is 1.175 or higher, it indicates that more oxygen is released than in normal cases, i.e., venous blood.

$$\text{Oxygen release rate} = A665/A880$$

Example 1

A spinning solution was obtained by dissolving 10 parts of an acrylonitrile polymer consisting of 90% acrylonitrile and 10% methyl acrylate in 90 parts of 48% aqueous solution of sodium rhodanate. Then, acrylic fiber A was obtained by spinning, drawing and drying according to the usual manner.
The acrylic fiber A was subjected to a crosslinking treatment in 15% hydrazine aqueous solution at 110°C for 3 hours and then washed with water. Next, the acrylic fiber A was subjected to an acid treatment in 8% nitric acid aqueous solution at 110°C for 1 hour and then washed with water. Subsequently, the acrylic fiber A was subjected to a hydrolysis treatment at 90°C in 5% sodium hydroxide aqueous solution and then washed with water. The amount of carboxyl groups in the fiber was adjusted by changing the hydrolysis treatment time. Thereafter, the acrylic fiber A was subjected to an acid treatment in 10% nitric acid aqueous solution at room temperature for 24 hours and then washed with water. In this way, an acid-type carboxyl group-containing material having a fibrous form was obtained.
The acid-type carboxyl group-containing material having a fibrous form and containing 5.2 mmol/g of acid-type carboxyl groups prepared as described above was mixed with the acrylic fiber A described above in a mass ratio of 1:99, and a nonwoven fabric sample was prepared from the resulting mixture by needle punching. The evaluation results of the sample are shown in Table 1.

Example 2

A nonwoven fabric sample was prepared in the same manner as in Example 1, except that the mass ratio of the acid-type carboxyl group-containing material having a fibrous form to the acrylic fiber A was changed to 5: 95. The evaluation results of the sample are shown in Table 1.

Example 3

A nonwoven fabric sample was prepared in the same manner as in Example 1, except that the mass ratio of the acid-type carboxyl group-containing material having a fibrous form to the acrylic fiber A was changed to 100:0. The evaluation results of the sample are shown in Table 1.

Example 4

A nonwoven fabric sample was prepared in the same manner as in Example 2, except that the amount of acid-type carboxyl groups in the acid-type carboxyl group-containing material having a fibrous form was changed from 5.2 mmol/g to 9.5 mmol/g. The evaluation results of the sample are shown in Table 1.

Example 5

A mixed aqueous solution containing 0.13% hydrazine and 35% sodium hydroxide was applied to the acrylic fiber A. Then, the acrylic fiber A was wrung in such a degree that the amount of liquid absorption relative to the fiber mass is 100% fiber absorption rate. The acrylic fiber A was then subjected to a crosslinking hydrolysis treatment at 106°C for 15 minutes and washed with water. The washed acrylic fiber A was then immersed in 0.1% sulfuric acid solution at 30°C for 1 hour, dehydrated, treated with an oil, dehydrated, opened, and dried. In this way, a sheath-core composite fiber consisting of a sheath portion and a core portion, wherein the sheath portion contains an acid-type carboxyl group-containing polymer, and wherein the core portion contains an acrylonitrile-based polymer was obtained. The resulting acid-type carboxyl group-containing material having a fibrous form and containing 1.8 mmol/g of acid-type carboxyl groups was mixed with the aforementioned acrylic fiber A in a mass ratio of 5:95, and a nonwoven fabric sample was prepared from the resulting mixture by needle punching. The evaluation results of the sample are shown in Table 1.

Comparative Example 1

A nonwoven fabric sample was prepared in the same manner as in Example 1, except that the mass ratio of the acid-type carboxyl group-containing material having a fibrous form to the acrylic fiber A was changed to 0:100. The evaluation results of the sample are shown in Table 1.

Comparative Example 2

A nonwoven fabric sample was prepared in the same manner as in Example 1, except that the amount of acid carboxyl groups in the acid carboxyl group-containing material having a fibrous form was changed from 5.2 mmol/g to 3.0 mmol/g. The evaluation results of the sample are shown in Table 1.

[Table 1]

|  | Example 1 | Example 2 | Example 3 | Example 4 | Example 5 | Comparative Example 1 | Comparative Example 2 |
|---|---|---|---|---|---|---|---|
| Amount of acid-type carboxyl groups in the material for promoting oxygen release [mmol/g] | 0.05 | 0.26 | 5.20 | 0.48 | 0.09 | 0.00 | 0.03 |
| pH in horse blood after treatment | 7.0 | 6.9 | 6.4 | 6.8 | 7.0 | 7.2 | 7.2 |

(continued)

| | Example 1 | Example 2 | Example 3 | Example 4 | Example 5 | Comparative Example 1 | Comparative Example 2 |
|---|---|---|---|---|---|---|---|
| Oxygen release rate | 1.281 | 1.283 | 1.335 | 1.286 | 1.281 | 1.164 | 1.170 |

As can be seen from Table 1, the oxygen release rate was good in each of the Examples of the present invention, and each of the Examples was useful as a material for promoting oxygen release. On the other hand, in each of Comparative Examples 1 and 2, the amount of acid-type carboxyl groups in the material for promoting oxygen release was small, and the oxygen release rate was not sufficient.

## Claims

1. A material for promoting oxygen release from red blood cells, **characterized in that** the material for promoting oxygen release from red blood cells is a sheet-like material containing an acid-type carboxyl group-containing material, wherein the amount of acid-type carboxyl groups contained in the sheet-like material is 0.05 mmol/g or more and 10.0 mmol/g or less.

2. The material for promoting oxygen release from red blood cells according to claim 1, wherein the amount of acid-type carboxyl groups in the acid-type carboxyl group-containing material is more than 1.5 mmol/g and not more than 10.0 mmol/g.

3. The material for promoting oxygen release from red blood cells according to claim 1, wherein the content of the acid-type carboxyl group-containing material is 1 to 100% by mass.

4. The material for promoting oxygen release from red blood cells according to claim 1, wherein the acid type carboxyl group-containing material has a fibrous, particulate or film form.

5. The material for promoting oxygen release from red blood cells according to claim 1, wherein the acid-type carboxyl group-containing material is a sheath-core composite fiber consisting of a sheath portion and a core portion, wherein the sheath portion contains an acid-type carboxyl group-containing polymer, and wherein the core portion contains an acrylonitrile-based polymer.

6. The material for promoting oxygen release from red blood cells according to any one of claims 1 to 5, wherein the material for promoting oxygen release from red blood cells is in the form of nonwoven fabric, woven fabric, knitted fabric, paper or film.

7. A wound dressing containing the material for promoting oxygen release from red blood cells according to claim 6.

8. The wound dressing according to claim 7, wherein a water vapor permeable material is laminated to the material for promoting oxygen release from red blood cells.

9. The wound dressing according to claim 8, wherein the water vapor permeable material is in the form of silicone or polyurethane film.

## INTERNATIONAL SEARCH REPORT

International application No.

**PCT/JP2024/022052**

| | |
|---|---|
| **A.** | **CLASSIFICATION OF SUBJECT MATTER** |

*A61L 15/24*(2006.01)i; *A61F 13/02*(2024.01)i; *A61K 9/70*(2006.01)i; *A61K 47/32*(2006.01)i; *A61K 47/34*(2017.01)i; *A61P 17/02*(2006.01)i

FI: A61L15/24 100; A61F13/02 310D; A61P17/02; A61K9/70 401; A61K47/32; A61K47/34

According to International Patent Classification (IPC) or to both national classification and IPC

| | |
|---|---|
| **B.** | **FIELDS SEARCHED** |

Minimum documentation searched (classification system followed by classification symbols)

A61L15/24; A61F13/02; A61K9/70; A61K47/32; A61K47/34; A61P17/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| Y | JP 2022-1266 A (HOLLISTER INCORPORATED) 06 January 2022 (2022-01-06) claims 4, 7, etc. claims, paragraphs [0011], [0016], [0029], examples, tables 1, 2 | 1-9 |
| Y | JP 7-216730 A (JAPAN EXLAN CO., LTD.) 15 August 1995 (1995-08-15) claims 1, paragraph [0038], examples, tables 1, 2 | 1-9 |
| Y | JP 10-237126 A (JAPAN EXLAN CO., LTD.) 08 September 1998 (1998-09-08) claims 1, 5, 10, 11, 13, etc. claims, [0039], [0042], table 1 examples 1-3 | 1-9 |
| Y | JP 2018-29643 A (NIPPON ELECTRIC GLASS CO.) 01 March 2018 (2018-03-01) paragraph [0004] | 1-9 |

✓ Further documents are listed in the continuation of Box C.      ✓ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **28 August 2024** | **10 September 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**EP 4 674 442 A1**

**INTERNATIONAL SEARCH REPORT**

| | International application No. |
|---|---|
| | **PCT/JP2024/022052** |

## C. DOCUMENTS CONSIDERED TO BE RELEVANT

| Category* | Citation of document, with indication, where appropriate, of the relevant passages | Relevant to claim No. |
|---|---|---|
| Y | 高岡駅南クリニック, 第03回　閉鎖環境とハイドロコロイドドレッシング材. 高岡駅南クリニックWebページ [online], 2021, [retrieved on: 27 August 2024], Internet, URL<https://web.archive.org/web/20210920231140/https://www.ekinan-clinic.com/publication/100>, (TAKAOKA EKINAN CLINIC), non-official translation (Part 3: Closed environment and hydrocolloid dressing materials. TAKAOKA EKINAN CLINIC Website.)<br>section "The wound healing promotion effect in a weakly acidic environment" | 1-9 |
| Y | 古田勝経, 褥瘡の保存的治療にかかわる薬剤師のために　その2　褥瘡の病態と局所環境における薬剤の特性, APJHP. 2005, vol. 33, no. 3, pp. 6-16, (FURUTA, Yoshinori), non-official translation (For pharmacists involved in conservative pressure injury treatment. Part 2: Pathological condition of pressure injury and characteristics of drugs in the local environment.)<br>p. 12, right column, section "(6) pH" | 1-9 |
| Y | JP 2001-17527 A (TOYO BOSEKI KABUSHIKI KAISHA) 23 January 2001 (2001-01-23)<br>paragraph [0025] | 8,9 |
| Y | JP 7-112021 A (TERUMO KABUSHIKI KAISHA) 02 May 1995 (1995-05-02)<br>paragraph [0020] | 8,9 |
| Y | WO 2004/073566 A1 (MENICON CO., LTD.) 02 September 2004 (2004-09-02)<br>description, p. 4, line 25 to p. 5, line 6 | 8,9 |

Form PCT/ISA/210 (second sheet) (July 2022)

## INTERNATIONAL SEARCH REPORT
### Information on patent family members

International application No.

**PCT/JP2024/022052**

| Patent document cited in search report | | | Publication date (day/month/year) | Patent family member(s) | Publication date (day/month/year) |
|---|---|---|---|---|---|
| JP | 2022-1266 | A | 06 January 2022 | WO 2016/028297 A1 <br> claims 6, 19, etc. claims, <br> paragraphs [0011], [0016], <br> [0027], examples, tables 1, 2 <br> US 2015/0045711 A1 <br> EP 3182946 A1 <br> CN 106714744 A | |
| JP | 7-216730 | A | 15 August 1995 | (Family: none) | |
| JP | 10-237126 | A | 08 September 1998 | (Family: none) | |
| JP | 2018-29643 | A | 01 March 2018 | (Family: none) | |
| JP | 2001-17527 | A | 23 January 2001 | (Family: none) | |
| JP | 7-112021 | A | 02 May 1995 | (Family: none) | |
| WO | 2004/073566 | A1 | 02 September 2004 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10201838 A
- JP 2000210375 A
- JP 2009173690 A
- JP 2013147774 A
- JP 2000314082 A
- JP 2023067820 A
- JP 2013147473 A

**Non-patent literature cited in the description**

- *Aichi Prefectural journal of hospital pharmacy*, 2005, vol. 33 (3), 6-16